# EUROPEAN PATENT APPLICATION

(11) **EP 2 468 749 A1**
(43) Date of publication of application: **27.06.2012**
(21) Application number: 11193941.9
(22) Date of filing: 16.12.2011
(51) Int. Cl.: C07D 473/04

(54) **Process for the preparation of Linagliptin**

(30) Priority: 23.12.2010 IT MI20102390
(71) Applicant: Dipharma Francis S.r.l., 20021 Baranzate (MI) (IT)
(72) Inventor: Allegrini, Pietro, 20021 Baranzate (MI) (IT); Attolino, Emanuele, 20021 Baranzate (MI) (IT); Artico, Marco, 20021 Baranzate (MI) (IT)
(74) Representative: Minoja, Fabrizio

(57) **Abstract**

The present invention relates to processes for the preparation of 8-(3R)-3-aminopiperidinyl)-7-butyn-2-yl-3-methyl-1-(4-methyl-quinazolin-2-ylmethyl)-3,7-dihydropurine-2,6-dione and novel intermediates useful in its synthesis.

## Description

### FIELD OF THE INVENTION

The present invention relates to processes for the preparation of 8-(3R)-3-aminopiperidinyl)-7-butyn-2-yl-3-methyl-1-(4-methyl-quinazolin-2-ylmethyl)-3,7-dihydropurine-2,6-dione and novel intermediates useful in its synthesis.

### TECHNOLOGICAL BACKGROUND

Linagliptin, namely 8-(3R)-3-aminopiperidinyl)-7-butyn-2-yl-3-methyl-1-(4-methylquinazolin-2-ylmethyl)-3,7-dihydropurine-2,6-dione, of formula (A), is a long acting inhibitor of dipeptidylpeptidase-IV (DPP-IV) activity, at present under development for the treatment of type II diabetes mellitus.

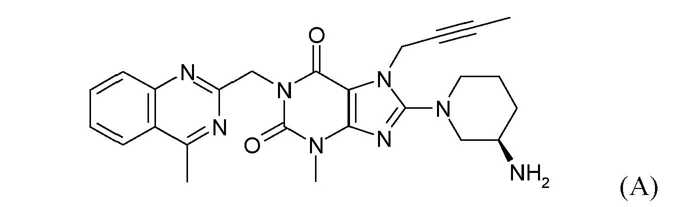

The synthesis of Linagliptin is reported in US 7,407,955, according to the scheme below, where 8-bromo xanthine of formula (B) is condensed with 3-(R)-Boc-aminopiperidine of formula (C) to obtain a compound of formula (D), which is converted to Linagliptin (A) by deprotection of the amine function

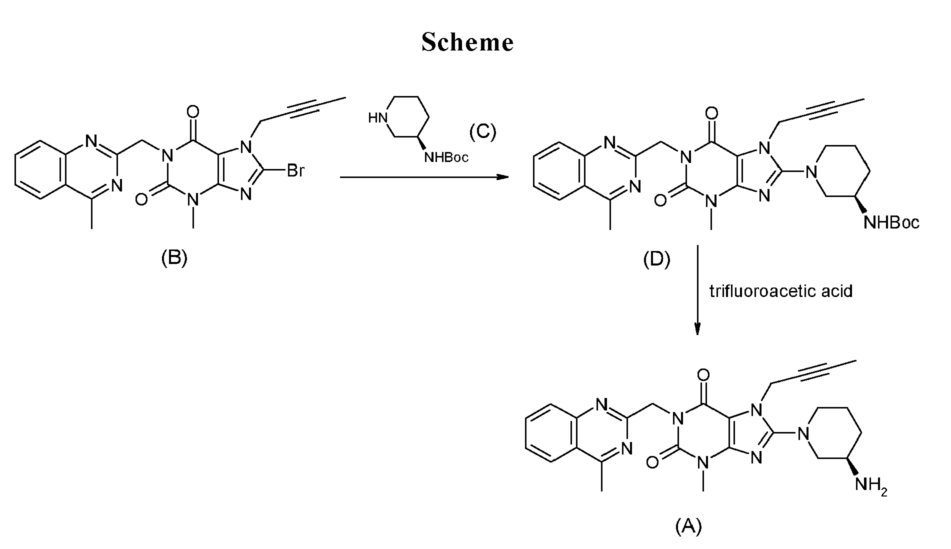

Optically active 3-aminopiperidine protected as the tert-butylcarbamate (Boc), compound (C), although commercially available, is very expensive and difficult to prepare; moreover in this process impurities are very difficult to remove, particularly on an industrial scale, in particular because of the Boc protective group. For this reason, US 2009/0192314 discloses a novel process for the preparation of Linagliptin (A) which makes use of a 3-(R)-aminopiperidine protected as a phthalimide of formula (E).

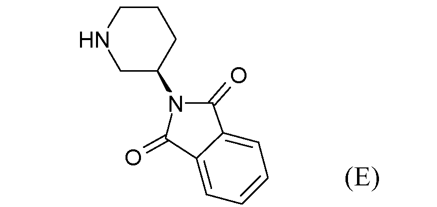

Accordingly, a compound of formula (E) can be prepared starting from 3-aminopyridine by hydrogenation, reaction with phthalic anhydride, resolution through diastereoisomeric salts using expensive D-tartaric acid, and then cleavage of the tartrate salt.

This intermediate is, however, still expensive and its use in the substitution reaction of the bromine derivative of formula (B) is still poorly efficient, as it takes place under drastic reaction conditions.

As it can be noted, these processes make use of drastic reaction conditions, or expensive, difficult to prepare starting materials, thus negatively affecting costs. There is therefore the need for an alternative synthetic route to provide Linagliptin or a salt thereof with high enantiomeric and chemical purity, from low cost starting materials.

### SUMMARY OF THE INVENTION

A novel process has now been found which allows to obtain Linagliptin or a salt thereof starting from low cost starting materials and with safe, reproducible procedures. The process of the invention is advantageous over those of the prior art.

### DETAILED DISCLOSURE OF THE INVENTION

Object of the invention is a process for the preparation of a compound of formula (**I**) or a salt thereof, in the anhydrous or hydrated form, either as a single (R) or (S) enantiomer, or as a mixture thereof, 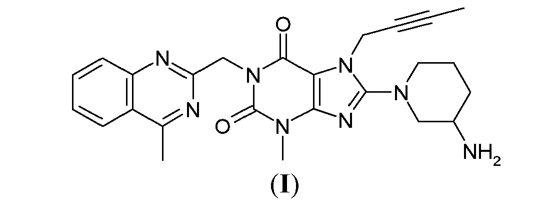 comprising the conversion of a compound of formula (**II**) or a salt thereof, either as a single enantiomer, or as a mixture thereof, 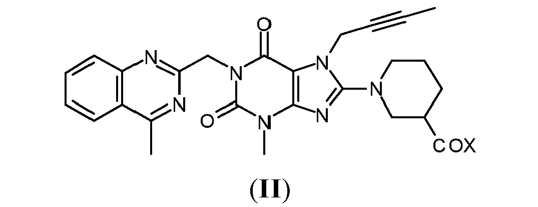 wherein X is hydrogen, hydroxy, C₁-C₈ alkyl, C₁-C₄ alkoxy, aryl, amino, N₃ or halogen; to a compound of formula (**I**) and, if desired, separation of a single enantiomer of formula (**I**) from an enantiomeric mixture, and/or, if desired, conversion of a compound of formula (**I**) to a salt thereof, and/or, if desired, conversion of a salt of a compound of formula (**I**) to the free base.

An enantiomer of a compound of formula (**I**) or (**II**) is preferably in absolute configuration (R).

A C₁-C₈ alkyl group, which can be straight or branched, is for example a C₁-C₆ alkyl group, preferably a C₁-C₄ alkyl group, particularly methyl, ethyl or isopropyl.

A C₁-C₄ alkoxy group, which can be straight or branched, is for example methoxy, ethoxy or isopropoxy.

An aryl group is for example phenyl or naphthyl, preferably phenyl.

A halogen is for example chlorine or bromine.

A salt of a compound of formula (**I**) is typically a pharmaceutically acceptable salt, for example the hydrochloride, trifluoroacetate or acetate, in the anhydrous or hydrated form. Preferably, the salt of a compound of formula (I) is the acetate salt.

The conversion of a compound of formula (II) to a compound of formula (I), as defined above, is preferably carried out through formation of an isocyanate compound.

For example, a compound of formula (II) in which X is N₃ can be converted to a compound of formula (I) by Curtius rearrangement, to obtain an isocyanate or carbamate intermediate, and subsequent hydrolysis to give a compound of formula (I).

A compound of formula (II) in which X is hydroxy can be converted to another compound of formula (II) in which X is N₃ by treatment with diphenylphosphorylazide (DPPA), preferably in presence of a base for example triethylamine, then subjected to Curtius rearrangement, to obtain an isocyanate or carbamate intermediate, and subsequent hydrolysis to give a compound of formula (I).

A compound of formula (II) in which X is hydroxy can be converted to a compound of formula (I) by Lossen or Schmidt rearrangement to obtain an isocyanate intermediate, and subsequent hydrolysis to give a compound of formula (I).

A compound of formula (II) in which X is NH₂ can be converted to a compound of formula (I) by Hofmann degradation reaction.

In particular, according to a preferred aspect of the invention, a compound of formula (**II**) in which X is N₃, either as a single enantiomer or as a mixture or a salt thereof, can be converted to a compound of formula (**I**), either as a single enantiomer or as a mixture or a salt thereof, by a process comprising Curtius rearrangement to obtain an intermediate isocyanate of formula (**III**), either as single enantiomer, or a mixture thereof; 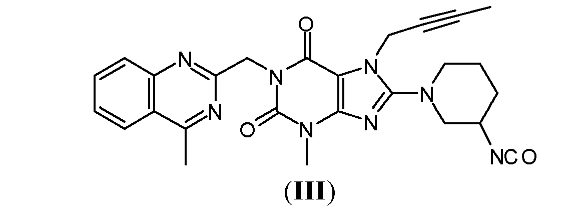 subsequent hydrolysis to give a compound of formula (**I**), and, if desired, separation of a single enantiomer of a compound of formula (**I**) from an enantiomeric mixture, and/or, if desired, conversion of a compound of formula (**I**) to a salt thereof, and/or *vice versa.*

Curtius rearrangement can be carried out in a solvent, selected from for example the group comprising a dipolar aprotic solvent, typically dimethylformamide, dimethylacetamide, acetonitrile or dimethylsulfoxide; an ether, typically tetrahydrofuran, dioxane or methyl-tert-butyl ether; a chlorinated solvent, typically dichloromethane; an apolar solvent, typically toluene; an ester, typically ethyl acetate, isopropyl acetate or butyl acetate; and a ketone, typically acetone, methyl ethyl ketone or methyl isobutyl ketone; or a mixture of two or more, preferably two or three, of said solvents. Preferably, Curtius rearrangement is carried out in acetonitrile.

After completion of the rearrangement, a compound of formula (**III**) can be isolated or not isolated.

The hydrolysis reaction is preferably carried out in the presence of water, at neutral or acid pH.

Alternatively, according to a further preferred aspect of the invention, the same Curtius rearrangement of a compound of formula (**II**) in which X is N₃, can be carried out in one of the solvents reported above or a mixture thereof, preferably acetonitrile, in the presence of water, at neutral or acid pH.

The intermediate isocyanate (**III**) in these conditions is hydrolysed to obtain the compound of formula (**I**).

Alternatively, according to a further preferred aspect of the invention, the same Curtius rearrangement of a compound of formula (**II**) in which X is N₃, can be carried out in the presence of an alcohol, preferably a Cᵢ-C₄ alkanol; an aliphatic or aromatic C₂-C₂₀ thiol, for example a C₂-C₂₀ alkyl-thiol; an aryl-thiol or an aryl-C₁-C₄-alkyl-thiol, to obtain a compound of formula (**IV**), either as a single (R) or (S) enantiomer or as a mixture thereof, 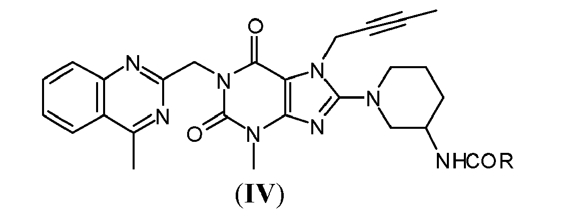 wherein R is a C₁-C₄ alkoxy group, or an aliphatic or aromatic C₂-C₂₀ thiol residue, for example a C₂-C₂₀ alkylthio, arylthio or aryl-C₁-C₄ alkylthio group; followed by hydrolysis to give a compound of formula (**I**), and if desired, separation of a single enantiomer of formula (**I**) from an enantiomeric mixture thereof, and/or, if desired, conversion of a compound of formula (**I**) to a salt thereof, or *vice versa.*

A C₁-C₄ alkanol, which can be straight or branched, is preferably isopropanol or tert-butanol.

A C₂-C₂₀ alkyl-thiol, which can be straight or branched, is for example dodecanethiol.

The aryl residue R in an arylthio or aryl-C₁-C₄ alkylthio group can be for example phenyl or naphthyl, or an unsaturated monocyclic or bicyclic heterocycle containing 1 to 3 heteroatoms independently selected from oxygen, sulfur and nitrogen. An aryl-thiol is preferably thiophenol or mercaptobenzimidazole.

More preferably a thiol, as exemplified above, is dodecanethiol or 2-mercaptobenzimidazole.

Curtius rearrangement can be carried out in one or more solvents, preferably one, two or three solvents selected from those indicated above, preferably acetonitrile.

After completion of the rearrangement, a compound of formula (**IV**) can be isolated or not isolated.

Hydrolysis of a compound of formula (**IV**) can be carried out according to known methods, in alkali or acid medium, for example using a base aqueous solution, optionally in the presence of a water- miscible or immiscible organic cosolvent, or by treatment with an acid aqueous solution, optionally in the presence of a water- miscible or immiscible organic cosolvent.

A base can be for example a hydroxide, carbonate or phosphate of an alkali metal, preferably sodium or potassium.

An acid can be for example a mineral acid, typically hydrochloric acid, or an organic acid, typically trifluoroacetic acid.

An organic cosolvent can be for example a solvent selected from the group consisting of a dipolar aprotic solvent, typically dimethylformamide, dimethylacetamide, N-methylpyrrolidone, acetonitrile or dimethylsulfoxide; an ether, typically tetrahydrofuran, dioxane or methyl-tert-butyl ether; a chlorinated solvent, typically dichloromethane; an apolar solvent, typically toluene; a polar protic solvent, preferably a C₁-C₄ alkanol; a ketone, typically acetone, methyl ethyl ketone or methyl isobutyl ketone; or a mixture of two or more, preferably two or three, of said solvents.

Preferably the cosolvent is a dipolar aprotic solvent, more preferably N-methylpyrrolidone.

Both Curtius rearrangement of a compound of formula (II) in which X is N₃, and the hydrolysis reactions, described above, can be carried out at a temperature ranging from about 20°C to the reflux temperature of the solvent.

If desired, a compound of formula (II) can be converted to another compound of formula (II), according to known methods, for example as reported hereinbelow, and then converted to a compound of formula (I), according to the process of the invention.

By way of example, according to a preferred aspect of the invention, a compound of formula (II) wherein X is hydroxy can be converted to a respective compound of formula (**II**) in which X is N₃ by treatment with diphenylphosphorylazide (DPPA), preferably in presence of a base for example triethylamine, then subjected to Curtius rearrangement and subsequent hydrolysis, according to the above reported procedures. Said conversion can be carried out in two separate steps or, preferably, in a single step (one-pot reaction).

According to a preferred aspect of the invention, a compound of formula (**II**) in which X is hydroxy, either as single enantiomer, or a mixture thereof, or a salt thereof, can be converted to a compound of formula (**I**), either as single enantiomer, or a mixture thereof or a salt thereof, by a process comprising Lossen or Schmidt rearrangement, to obtain an intermediate isocyanate of formula (**III**), as defined above, either as single enantiomer, or a mixture thereof; subsequent hydrolysis of (**III**) according to what reported above and, if desired, separation of a single enantiomer of formula (**I**) from its racemic mixture, and/or, if desired, conversion of a compound of formula (**I**) to a salt thereof, or *vice versa.*

The Lossen or Schmidt rearrangement can be carried out in agreement to procedures known in the art.

According to a preferred aspect of the invention, a compound of formula (**II**) in which X is NH₂, either as a single enantiomer or as an enantiomeric mixture or a salt thereof, can be converted to a compound of formula (**I**), either as single enantiomer, or a mixture thereof, or a salt thereof, by a process comprising Hofmann degradation and, if desired, separation of a single enantiomer of formula (**I**) from its racemic mixture, and/or, if desired, conversion of a compound of formula (**I**) to a salt thereof, or *vice versa.*

The reaction can be carried out by treatment with a reagent capable of providing halogen ions in oxidation state (**I**), preferably hypobromite or hypochlorite ions.

Said reagent is for example an hypobromite or hypochlorite salt with a cation of an alkali metal, preferably sodium or potassium, or with an organic cation such as an alkylammonium, for example tetrabutylammonium. Hypochlorite and hypobromite can be prepared by dissolving molecular chlorine or bromine in a suitable basic aqueous solution, or using reagents capable of developing molecular chlorine or bromine *in situ,* in an alkali medium, for example N-bromosuccinimide.

If desired, Hofmann reaction can be carried out using a hypobromite or hypochlorite aqueous solution, as defined above, made basic by the presence of a hydroxide an alkali metal, for example sodium or potassium.

If desired, Hofmann reaction can be carried out using a basic alcoholic solution, for example for the presence of a tertiary amine or an alkali metal C₁-C₄ alkoxide, and recovering the intermediate carbamate of formula (**IV**), as defined above, wherein R is a C₁-C₄ alkoxy group, which is then subjected to basic hydrolysis to yield compound of formula (I), in optically active form as a single enantiomer or as an enantiomeric mixture.

An alkali metal C₁-C₄ alkoxide is preferably a sodium or potassium salt, for example sodium or potassium methoxide or ethoxide.

A tertiary amine can be for example 1,8-diazabicyclo[5.4.0]undec-7-ene (DBU), 1,5-diazabicyclo[4.3.0]non-5-ene (DBN), or 1,4-diazabicyclo[2.2.2]-octane (DABCO).

The separation of a single enantiomer of a compound of formula (**I**), or a salt thereof, for example the individual isomer in (R) or (S) optically active form, from a mixture of its enantiomers, can be carried out according to known methods, for example through resolution by use of an optically active acid, typically tartaric, mandelic or camphorsulfonic acids.

The conversion of a compound of formula (**I**) to a salt thereof, or the conversion of a salt thereof to the free base, can be obtained according to known methods.

A resulting compound of formula (**I**), in particular with absolute configuration (R), i.e. Linagliptin, has chemical purity equal to or higher than 99%, in particular equal to or higher than 99.9%.

Linagliptin with such purity degree can be used for the preparation of a salt thereof, for example the hydrochloride, having the same purity degree.

The enantiomeric purity of Linagliptin, as obtained according to the invention, is equal to or higher than 99.0%. Said purity can be optionally increased for example up to 99.9%, by known techniques, for example by crystallization.

The size of Linagliptin crystals, as obtainable according to present invention, is characterized by a D₅₀ value approximately ranging from 10 to 250 µm. If desired, said value can be reduced by micronisation or fine grinding.

The conversion of an optically active compound of formula (**II**) with (R) or (S) configuration, having chemical and enantiomeric purities equal to or higher than 99%, according to the process of the invention, provides the optically active compound of formula (**I**) with (R) or (S) configuration, in extremely high purity degree, both from the chemical and stereochemical point of view.

A compound of formula (**II**) in which X is C₁-C₄ alkoxy, either as single enantiomer, or a mixture thereof, can be prepared by a process comprising the reaction of a compound of formula (**VI**) 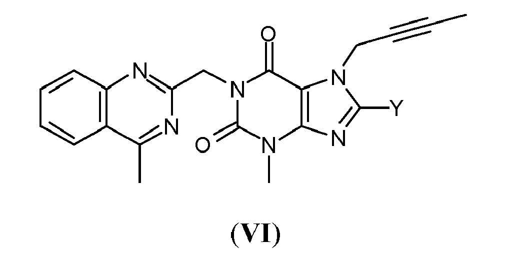 wherein Y is a leaving group, preferably halogen, for example chlorine or bromine, more preferably bromine, with a compound of formula (**V**), either as single enantiomer, or a mixture thereof, or a salt thereof, 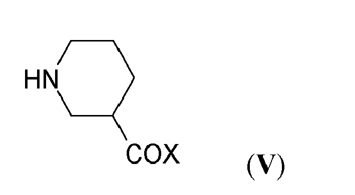 wherein X is C₁-C₄-alkoxy, in the presence of a base, and optionally of a solvent.

A salt of a compound of formula (**V**) is preferably a pharmaceutically acceptable salt.

A compound of formula (**V**) has preferably absolute configuration (R).

A base can be for example organic or inorganic, weak or strong. An organic base can be for example a C₁-C₄ alkoxide of an alkali metal, preferably sodium; or a tertiary amine, for example triethylamine, diisopropylethylamine, 1,8-diazabicyclo[5.4.0]undec-7-ene (DBU), 1,5-diazabicyclo[4.3.0]non-5-ene (DBN), 1,4-diazabicyclo[2.2.2]octane (DABCO); lithium diisopropilamide (LDA); isopropyl-magnesium bromide or chloride; cyclohexyl-magnesium bromide or chloride; a C₄-C₆ alkyl-lithium or phenyl-lithium. An inorganic base can be a carbonate, phosphate, mono or dihydrogen phosphate, or a hydroxide of an alkali or alkaline-earth metal, preferably sodium or potassium.

Preferably the base is triethylamine or diisopropylethylamine.

The reaction of a compound of formula (**V**) with a compound of formula (**VI**) can be carried out in a solvent, selected for example from the group comprising a dipolar aprotic solvent, typically dimethylformamide, dimethylacetamide, N-methylpyrrolidone, acetonitrile or dimethylsulfoxide; an ether, typically tetrahydrofuran or dioxane; a chlorinated solvent, typically dichloromethane; an apolar solvent typically toluene; an ester, typically ethyl acetate, isopropyl acetate or butyl acetate; a ketone, typically acetone, methyl ethyl ketone or methyl isobutyl ketone; or a mixture of two or more, preferably two or three, of said solvents.

Preferably, the reaction is carried out in a dipolar aprotic solvent, more preferably in N-methylpyrrolidone.

A compound of formula (II), as defined above, can be converted to another compound of formula (II), according to known methods.

For example, a compound of formula (II), as defined above, in which X is hydroxy, or a salt thereof, as a single enantiomer or as a mixture thereof, can be prepared by hydrolysis of a corresponding compound of formula (II) in which X is C₁-C₄ alkoxy.

Hydrolysis of an alkyl ester of formula (II) can be carried out for example by treating a solution thereof in a C₁-C₄ alkanol, for example methanol or ethanol, with an aqueous solution of an alkali metal hydroxide, preferably sodium or potassium, then adjusting the resulting mixture to acid pH. The hydrolysis can be preferably carried out under heating at a temperature ranging from about 25°C to the reflux temperature of the reaction mixture.

A thus prepared compound of formula (II) in which X is hydroxy, can be in the form of a single (R) or (S) enantiomer, or of a mixture thereof, typically a racemic mixture.

A compound of formula (II) in which X, being as defined above, is different from hydroxy, can be prepared from a compound of formula (II) wherein X is hydroxy, according to known methods.

For example, a compound of formula (II), as defined above, wherein X is C₁-C₄ alkoxy, can be obtained from a compound of formula (II) wherein X is hydroxy by esterification with a C₁-C₄ alkanol, according to known methods.

A compound of formula (II) in which X is N₃ either as single enantiomer, or a mixture thereof, can be obtained from a compound of formula (II) wherein X is hydroxy, by treatment with diphenylphosphorylazide (DPPA), preferably in the presence of a base as defined above. Said compound of formula (II) can be isolated or used as such in the subsequent Curtius rearrangement.

A compound of formula (II), or a salt thereof, either as single enantiomer, or a mixture thereof, is a novel compound and is a further object of the present invention. Preferred compounds of the invention are those of formula (II) in which X is hydroxy, in particular those with absolute configuration (R).

A compound of formula (VI) can be prepared for example according to what reported in US 7,407,955. A compound of formula (V), either as a single (R) or (S) enantiomer, or as a mixture thereof, typically a racemic mixture, and the salts thereof, is commercially available, or can be prepared with simple, inexpensive techniques according to what reported in Chirality 1995, 7, 90-95.

In particular, a compound of formula (V) wherein X is ethoxy, with absolute configuration (R), and enantiomeric purity equal to or higher than 99.5%, therefore useful as an intermediate in the preparation of a compound of formula (I), as a single enantiomer (R), namely Linagliptin, can be obtained from inexpensive ethyl nicotinate of formula (VII) 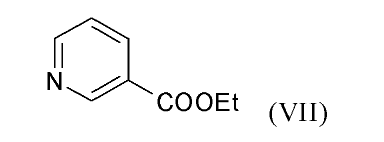 upon hydrogenation and resolution of the diastereomeric salts thereof, obtained by reaction with (L) tartaric acid, in agreement to known techniques.

A resulting compound of formula (II), as a single (R) or (S) enantiomer, has chemical and enantiomeric purities equal to or higher than 99%, and its conversion to a compound of formula (I), according to the process of the invention, provides a compound of formula (I) in extremely high purity degree, both from the chemical and stereochemical point of view.

The following examples illustrate the invention.

### Example 1: Preparation of a compound of formula (II) with X=OEt

The bromoxanthine of formula (B) prepared according to US 7,407, 955 (28.2 g, NMR title 90%, 56.0 mmols) and L-(+)-tartrate salt of (R)-ethylnipecotate (22.4 g, 72.8 mmols) are suspended in 50 mL of 1-methyl-2-pyrrolidone. The suspension is heated at 100° under stirring and, maintaining such temperature, diisopropylethylamine (38.3 ml, 224 mmols) is slowly dropwise added. The suspension is moderately refluxed for 2 hours. The mixture is cooled to 30°C and 400 mL of are dropwise added under vigorous stirring. The suspension is stirred for 30 minutes, then filtered off and the solid is washed with 100 mL of water. 27 g of solid product are obtained after drying with a 90% yield.

¹H-NMR (300 MHz, CDCl₃), δ 8.02 (d, 1H), 7.87 (d, 1H), 7.76 (t, 1H), 7.51 (t, 1H), 5.55 (s, 2H), 4.90 (s, 2H), 4.25 - 4.10 (m, 2H), 3.82 (dd, 1H), 3.65 - 3.51 (m, 4H), 3.33 (dd, 1H), 3.15 (m, 1H), 2.88 - 2.72 (m, 4H), 2.08 (m, 1H), 1.92 - 1.73 (m, 6H), 1.27 (t, 3H).

### Example 2: Preparation of a compound of formula (II) with X=OH

The compound of formula (II) having X = OEt, prepared according to Example 1 (27 g, 51 mmols), is suspended in 270 mL of MeOH and 4.1 g of NaOH scales and 13.7 mL of water are added under stirring. The reaction mixture is maintained under stirring for 2 hours at reflux temperature and then cooled to 40°C and diluted with 400 ml of water.

The mixture is then acidified by adding 6.6 mL of acetic acid and the solid is filtered off and washed with water and dried under vacuum at 50°C, obtaining 21 g of product, with a yield of 82%.

¹H-NMR (300 MHz, DMSO-d₆), δ 8.11 (d, 1H), 7.85 (t, 1H), 7.80 (d, 1H), 7.62 (t, 1H), 5.30 (s, 2H), 4.87 (s, 2H), 3.79 (dd, 1H), 3.57 (m, 1H), 3.38 (s, 3H), 3.33 (dd, 1H), 3.10 (m, 1H), 2.85 (s, 3H), 2.62 (m, 1H), 1.95 (m, 1H), 1.78 - 1.60 (m, 6H).

### Example 3: Preparation of a compound of formula (IV) with R = OCH(CH₃)₂

The compound of formula (II) with X=OH prepared according to Example 2 (0.5 g; 1 mmol), 5 ml of isopropanol and trietylamine (0.17 ml, 1.2 mmols) are mixed under stirring. 0.3 g of diphenylphosphorylazide (DPPA) are added in a sole portion. The mixture is heated at reflux temperature for 2 hours under stirring. The mixture is then cooled to room temperature and the solid is filtered off and washed with 2 ml of isopropyl alcohol. The solid is dried under vacuum at 50°C obtaining 0.4 g of product with a yield of 72%.

¹H-NMR (300 MHz, DMSO-d₆), δ 8.12 (d, 1H), 7.85 (t, 1H), 7.80 (d, 1H), 7.63 (t, 1H), 5.28 (s, 2H), 4.85 (s, 2H), 4.75 (ep, 1H), 4.27 (d, 1H), 3.78-3.55 (m, 4H), 3.35 (s, 3H), 2.85 (s, 3H), 1.85 - 1.60 (m, 6H). 1.42 (m, 1H), 1.02 (d, 6H).

### Example 4: Preparation of Linagliptin

The carbamate of formula (IV), prepared according to Example 3 (400 mg, 0.72 mmols), is dissolved in 5 ml of 32% HCl in water. The reaction mixture is maintained under stirring at 65-70°C for 7 hours and then cooled to room temperature. The pH of the solution is brought to about 8-9 by treatment with 30% NaOH in water and the obtained suspension is stirred for 10 minutes and then filtered off. The solid is dissolved in 10 ml of AcOEt, the solution is filtered and the filtrate is evaporated under reduced pressure. 250 mg of Linagliptin are obtained with a yield of 73%.

### Example 5: Preparation of a compound of formula (IV) with R = S(CH₂)₁₁CH₃

The compound of formula (II) with X =OH, prepared according to Example 2 (3.0 g, 6 mmols), 30 ml of acetonitrile and triethylamine (1.09 ml, 7.8 mmols) are mixed together. Subsequently, 1.55 ml (7.2 mmols) of diphenylphosphorylazide (DPPA) are added. The reaction mixture is heated at reflux temperature for 1 hour under stirring and then cooled to 60°C and treated with dodecanethiol (1.87 ml, 7.8 mmols). The mixture is maintained under stirring at the same temperature for 30 minutes and then cooled to 25°C. The formed solid is filtered off and washed with 10 ml of acetonitrile. The solid is dried under vacuum at 60°C obtaining 3.5 g of product with a yield of 85%.

¹H-NMR (300 MHz, DMSO-d₆), δ 8.21 (d, 1H), 7.88 (t, 1H), 7.83 (d, 1H), 7.64 (t, 1H), 5.30 (s, 2H), 4.86 (s, 2H), 3.85 (m, 1H), 3.70 (d, 1H), 3.56 (d, 1H), 3.38 (s, 3H), 3.10-2.87 (m, 3H), 2.85 (s, 3H), 2.74 (t, 2H), 1.90-1.60 (m, 3H), 1.74 (s, 3H), 1.60-1.40 (m, 2H), 1.38-1.10 (m, 18H), 0.82 (t, 3H).

### Example 6: Preparation of Linagliptin

The thiocarbamate of formula (IV) (10 g, 14,3 mmols), prepared according to Example 5, is dissolved in 100 mL of N-methylpyrrolidone (NMP) and treated with a 30% NaOH solution (7.6 g, 57.0 mmols). The reaction mixture is stirred for 3 hours and then diluted with water and acidified by adding concentrated H₂SO₄. The mixture is extracted with hexane and brought to pH 9.5 by adding 30% NaOH and repeatedly extracted with dichloromethane. The dichloromethane phases are collected and washed with water and then dried over Na₂SO₄, filtered and concentrated under reduced pressure. The so obtained oily residue is then dissolved in methyl tert-butyl ether (MTBE) and the mixture is maintained under stirring for 2 hours, then cooled to 0-5°C and the so obtained solid is filtered off, washed with MTBE and dried under vacuum at 50°C till constant weight. 4.2 g of Linagliptin with a yield of 63% are obtained.

### Example 7: Preparation of a compound of formula (IV) with R=C₇H₅N₂S (2-mercaptobenzoimidazole)

The compound of formula (II) with X =OH, prepared according to Example 2 (2.0 g, 4 mmols), 20 ml of acetonitrile and triethylamine (0.8 ml, 5.6 mmols) are mixed together. Subsequently, 1.43 g (5.2 mmols) of diphenylphosphorylazide (DPPA) are added. The reaction mixture is heated at reflux temperature for 1 hour under stirring and then cooled to 60°C and treated with 2-marcaptobenzimidazole (0.8 g, 5.2 mmols). The mixture is maintained under stirring at the same temperature for 30 minutes, then cooled to 25°C and evaporated under reduced pressure with Rotavapor®. The residue is treated with 50 ml of dichloromethane (CH₂Cl₂) and washed with 2X20 ml of 5% NaOH. The organic phase is dried over Na₂SO₄, filtered and concentrated under reduced pressure and the residue is triturated with 30 ml of MTBE. The so obtained solid is filtered off, dried under vacuum at 60°C till constant weight obtaining 2.5 g of light brown powder.

### Example 8: Preparation of Linagliptin

Starting from the compound of formula (**IV**) as obtained in example 7 and following the procedure of example 6, product Linagliptin is obtained.

## Claims

1. Process for the preparation of a compound having the following formula (**I**), or a salt thereof, in anhydrous or hydrate form, either as single (R) or (S) enantiomer, or a mixture thereof, 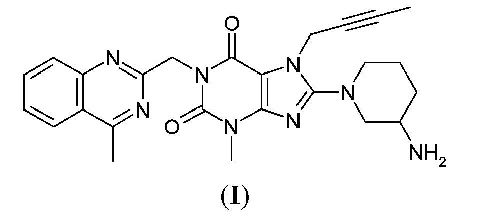 comprising converting a compound of formula (**II**), or a salt thereof, either as single enantiomer, or a mixture thereof, 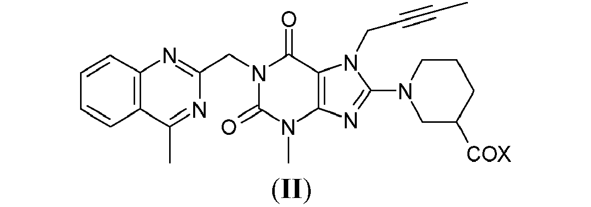 wherein X is hydrogen, hydroxy, C₁-C₈ alkyl, C₁-C₄ alkoxy, aryl, amino, N₃ or halogen; into a compound of formula (**I**), and, if desired, separating a single enantiomer of formula (**I**) from a mixture thereof, and/or, if desired, converting a compound of formula (**I**) into a salt thereof, and/or, if desired, converting a salt of a compound of formula (**I**) into the free base.

2. Process according to claim 1, wherein a compound of formula (**II**), wherein X is N₃, is converted into a compound of formula (**I**) by Curtius rearrangement, to obtain an isocyanate or carbamate intermediate, and its subsequent hydrolysis to obtain a compound of formula (**I**).

3. Process according to claim 1, wherein a compound of formula (**II**), wherein X is hydroxy, is converted into another compound of formula (**II**) wherein X is N₃ by treatment with diphenylphosphoryl azide (DPPA), and then submitted to a Curtius rearrangement to obtain an isocyanate or carbamate intermediate and then submitted to hydrolysis to obtain a compound of formula (**I**).

4. A process according to claims 2 or 3, wherein the Curtius rearrangement is carried out in the presence of an alcohol preferably a C₁-C₄ alkanol; an aliphatic or aromatic C₂-C₂₀ thiol, for example a C₂-C₂₀ alkyl-thiol; an aryl-thiol or an aryl-C₁-C₄-alkyl-thiol, to obtain a compound of formula (IV), either as a single (R) or (S) enantiomer or as a mixture thereof, 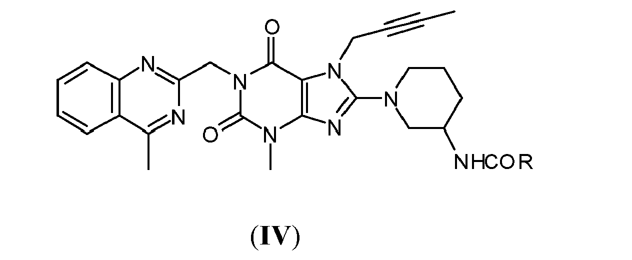 wherein R is a C₁-C₄ alkoxy group, or an aliphatic or aromatic C₂-C₂₀ thiol residue, for example a C₂-C₂₀ alkylthio, arylthio or aryl-C₁-C₄ alkylthio group; followed by hydrolysis to give a compound of formula (**I**), and if desired, separation of a single enantiomer of formula (**I**) from an enantiomeric mixture thereof, and/or, if desired, conversion of a compound of formula (**I**) to a salt thereof, or *vice versa.*

5. Process according to claim 1, wherein a compound of formula (**II**), wherein X is hydroxy, is submitted to a Lossen or Schmidt rearrangement to obtain an isocyanate intermediate and its subsequent hydrolysis to obtain a compound of formula (**I**).

6. Process according to claim 1 wherein a compound of formula (**II**) wherein X is NH₂ is converted into a compound of formula (**I**) by Hofmann degradation reaction.

7. A compound of formula (**II**) or a salt thereof, or a compound of formula (**III**) or of formula (**IV**), either as a single enantiomer or a mixture thereof, 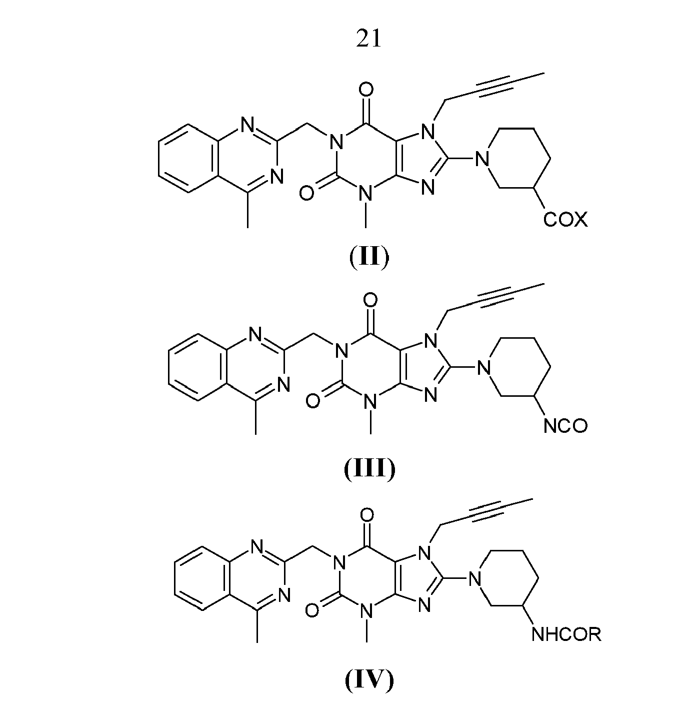 wherein X is hydrogen, hydroxy, C₁-C₈ alkyl, C₁-C₄ alkoxy, aryl, amino, N₃ or halogen; and
R is a C₁-C₄ alkoxy group, or an aliphatic or aromatic C₂-C₂₀ thiol residue, preferably C₂-C₂₀ alkylthio, arylthio, or aryl-C₁-C₄ alkylthio.

8. A compound of formula (**II**), or a salt thereof, as defined in claim 7, having (R) configuration;

9. A compound of formula (**II**), as defined in claim 7, with (R) or (S) configuration having chemical and enantiomeric purities equal to or higher than 99%.
